# EUROPÄISCHE PATENTSCHRIFT

(11) **EP 2 883 534 B1**
(45) Veröffentlichungstag und Bekanntmachung des Hinweises auf die Patenterteilung: **04.07.2018**
(21) Anmeldenummer: 14157680.1
(22) Anmeldetag: 04.03.2014
(51) Int. Cl.: A61K 8/34, A61Q 5/00

(54) **Wärmende 2K-Öl-Haarkur**
Warming 2-component oil hair treatment
Masque chauffant oléagineux bi-composants

(30) Priorität: 10.12.2013 DE 102013225370
(43) Veröffentlichungstag der Anmeldung: 17.06.2015
(73) Patentinhaber: Henkel AG & Co. KGaA, 40589 Düsseldorf (DE)
(72) Erfinder: Battermann, Marlene, 21271 Asendorf (DE); Hippe, Thomas, 25482 Appen (DE)

(56) Entgegenhaltungen:
- EP-A2- 0 027 730
- EP-A2- 1 226 812
- DE-A1- 2 317 140
- DE-U1- 9 211 006
- US-A1- 2001 016 201
- DATABASE GNPD [Online] MINTEL; 1. Juli 2013 (2013-07-01), "Self Warming Hair Mask", XP002739172, Database accession no. 2117022
- "Dove Hair Therapy Selbsterwärmende Haarmaske", INTERNET CITATION, 14. Juli 2013 (2013-07-14), Seiten 1-5, XP007923041, Gefunden im Internet: URL:http://genofevas-beauty.blogspot.com/2 013/07/dove-hair-therapy-selbsterwarmende. html [gefunden am 2015-03-06]
- Anonymous: "Dove Intensive Repair Self Warming Hair Mask - 15 ml: Amazon.co.uk: Beauty", , 17 December 2015 (2015-12-17), XP055237384, Retrieved from the Internet: URL:http://www.amazon.co.uk/Dove-Intensive -Repair-Self-Warming/dp/B00KDUVUDI [retrieved on 2015-12-18]

## Beschreibung

Die Erfindung betrifft Mittel zur Behandlung von keratinhaltigen Fasern, insbesondere menschlichen Haaren, welches in zwei getrennte verpackten Zubereitungen mindestens ein Öl sowie eine PEG-Verbindung enthält.

Pflegeprodukte für keratinische Fasern weisen oft den Nachteil auf, daß sie das Haar beschweren und damit dessen Fülle verringern. Dieses Problem tritt insbesondere bei Produkten auf, die im Haar belassen werden (so genannte leave-on-Produkte). Im Gegensatz dazu haben Produkte, die kurz nach ihrer Anwendung ausgespült werden (so genannte rinse-off-Produkte) oft nicht genügen Pflegepotential.

Im Handel sind selbsterwärmende Haarmasken, z.B. "Dove self warming hair mask", erhältlich, die aus zwei getrennt voneinander verpackten Zubereitungen bestehen. Eine dieser Zusammensetzungen ist eine Haarkur, welche Wasser, Öl und Kationtenside enthält, und die zweite Zubereitung enthält Polyethylenglycol. Beim Vermischen der beiden Zubereitungen entwickelt sich Wärme.

Haarkuren mit einer Zusammensetzung, die durch das Vermischen von zwei bis zur Anwendung voneinander getrennt gehaltenen Komponenten erhalten wurden, wobei eine der Komponenten Polyethylenglycole enthält und wobei durch das Vermischen Wärme entsteht, sind beispielsweise auch aus DE 92 11 006 U1, US2001/016201 A1 und EP 1 226 812 A2 bekannt. In der DE 23 17 140 A1 wird die Erwärmung durch exotherme Hydratation bewirkt.

Der vorliegenden Erfindung lag die Aufgabe zugrunde, Haarbehandlungsmittel bereitzustellen, die das Haar nachhaltig pflegen, die Kämmbarkeiten, Glanz und Griff verbessern, ohne dabei die Frisur zu beschweren. Idealerweise sollte dies mit Produkten erreicht werden können, die sowohl in relativ kurzer Zeit nach ihrer Applikation wieder vom Haar abgespült werden, als auch auf dem Haar verbleiben können.

Eine weitere Aufgabe der vorliegenden Erfindung bestand darin, dem Verbraucher das Pflegeerlebnis noch deutlicher zu machen, d.h. die Pflegeeffekte einerseits zu verstärken und andererseits mit einem für den Verbraucher wahrnehmbaren Gefühl zu unterstreichen.

In nicht vorhersehbarer Weise wurde nun gefunden, daß bestimmte ölhaltige Pflegemittel für Haare durch den Zusatz bestimmter PEG-Verbindungen in ihrer Pflegeleistung verstärkt werden können und darüber hinaus bei der Anwendung zu einer leichten angenehmen Erwärmung auf dem Kopf des Anwenders führen, was die erzielten Pflegeeffekte einerseits verbessert und andererseits auf einzigartige Weise erlebbar macht.

Gegenstand der vorliegenden Erfindung sind Mittel zur Pflege keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, wobei
- Zubereitung (A) eine Haarkur ist, die - bezogen auf ihr Gewicht -
   a) 10 bis 60 Gew.-% Wasser,
   b) 2,5 bis 20 Gew.-% Isopropylmyristat;
   c) 1 bis 10 Gew.-% kationische(s) Tensid(e)
   enthält,
   wobei Zubereitung (A) maximal 20 Gew.-% Öl(e) enthält
   und
- Zubereitung (B) eine fließfähige Zusammensetzung ist, die - bezogen auf ihr Gewicht - mindestens 50 Gew.-% Polyethylenglycol(e) der Formel (I) 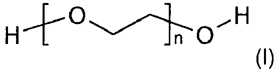 mit n = 6, 7, 8, 9 oder 10
   enthält.

Die erste Zubereitung (A) ist wasserbasiert. Der Begriff "wasserbasiert" wird erfindungsgemäß so verstanden, daß die Zusammensetzungen 10 bis maximal 60 Gew.-%, bevorzugt 20 bis maximal 55 Gew.-%, freies Wasser enthalten, bezogen auf die gesamte Zusammensetzung. Der Gehalt an Kristallwasser, Hydratationswasser oder ähnlich molekular gebundenem Wasser, der in den eingesetzten Bestandteilen enthalten sein kann, stellt im Sinne der vorliegenden Anmeldung kein freies Wasser dar.

Als wesentlichen Inhaltsstoff enthalten die Zubereitungen (A) 2,5 bis 20 Gew.-% Isopropylmyristat und insgesamt maximal 20 Gew.-% Öl(e).

Zu den natürlichen und synthetischen kosmetischen Ölen sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethylhexyl)-cyclohexan (Cetiol® S) und Di-n-octylether (Cetiol® OE) können bevorzugt sein.

Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von techni
schen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol® CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 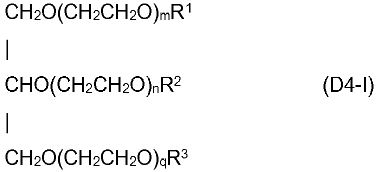 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, daß mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure,
   Linolensäure, Elaeostearinsäure, Arachinsäure, Gadoleinsäure, Behensäure und Erucasäure
   sowie deren technische Mischungen. Vorzugsweise werden Ölsäuremonoglyceride eingesetzt. Als natürliche Öle kommen beispielsweise Amaranthsamenöl, Aprikosenkernöl, Arganöl, Avocadoöl, Babassuöl, Baumwollsaatöl, Borretschsamenöl, Camelinaöl, Distelöl, Erdnußöl, Granatapfelkernöl, Grapefruitsamenöl, Hanföl, Haselnussöl, Holundersamenöl, Johannesbeersamenöl, Jojobaöl, Kakaobutter, Leinöl, Macadamianussöl, Maiskeimöl, Mandelöl, Marulaöl, Nachtkerzenöl, Olivenöl, Palmöl, Rapsöl, Reisöl, Sanddornfruchtfleischöl, Sanddornkernöl, Sesamöl, Sheabutter, Sojaöl, Sonnenblumenöl, Traubenkernöl, Walnußöl oder Wildrosenöl.

Die Zubereitung (A) kann als Öl auch flüssigen Carbonsäureester enthalten, die sich von C₂-C₈-Monoalkanolen mit einer Mono- oder Dicarbonsäure ableiten.

Beispiele für C₂-C₈-Monoalkanole sind Ethanol, n-Propanol, Isopropanol (1-Methylethanol), 1-Butanol, 2-Butanol, 2-Methylpropan-2-ol (tert-Butanol), 2-Methylpropan-1-ol (Isobutanol), 1-Pentanol, 2-Pentanol, 3-Pentanol, 3-Methylbutan-1-ol (Isopentanol), 3-Methylbutan-2-ol (Siamylalkohol), 2-Methyl-2-butanol, 2,2-Dimethylpropan-1-ol (Neopentylalkohol), 1-Hexanol, 4-Methylpentan-1-ol (Isohexanol), 1-Heptanol, 1-Octanol, 6-Methylheptan-1-ol, 3,3-Dimethylhexan-1-ol, 3,5-Dimethylhexan-1-ol, 4,5-Dimethylhexan-1-ol, 3-Methylheptan-1-ol u. 5-Methylheptan-1-ol (Isooctanole) und 2-Ethylhexan-1-ol (Ethylhexylalkohol). Beispiele für geeignete Monocarbonsäuren sind gesättigte Fettsäuren, wie Decansäure, Dodecansäure (Laurinsäure), Tetradecansäure (Myristinsäure), Hexadecansäure (Palmitinsäure), Octadecansäure (Stearinsäure) und 16-Methylheptadecansäure (Isostearinsäure), ungesättigte Fettsäuren wie Palmitoleinsäure (C16:1; 9*Z*), Ölsäure (C18:1; 9*Z*), Elaidinsäure (C18:1; 9*E*), Eicosensäure (Gondosäure; C20:1; 11*Z*), Linolsäure (C18:2; 9*Z*, 12*Z*), γ-(gamma)-Linolensäure (C18:3; 6*Z*, 9*Z*, 12*Z*), α-(alpha)-Linolensäure (C18:3; 9*Z*, 12*Z*, 15*Z*), α-Elaeostearinsäure (C18:3; 9*Z*, 11*E*, 13*E*) und Arachidonsäure (C20:4; 5*Z*, 8*Z*, 11*Z*, 14*Z*) sowie natürliche Fettsäureschnitte wie Fettsäuren aus Lanolin (Lanolate), Cocosfett (Cocoate) und Talgfett (Tallowate). Beispiele für geeignete Dicarbonsäuren sind gesättigte Dicarbonsäuren mit 4 bis 10 Kohlenstoffatomen, insbesondere Bernsteinsäure (Ethan-1,2-dicarbonsäure, Succinsäure), Glutarsäure (Propan-1,3-dicarbonsäure), Adipinsäure (Butan-1,4-dicarbonsäure) und Sebacinsäure (Oktan-1,8-dicarbonsäure). Bevorzugt sind dabei solche Carbonsäureester, welche über eine Gesamtkohlenstoffanzahl von 12 bis 22 Kohlenstoffatomen verfügen. Bevorzugte Verbindungen sind Ethyllaurat, Propyllaurat, Isopropyllaurat (IPL), Butyllaurat, Hexyllaurat, Ethylhexyllaurat, Ethylmyristat, Propylmyristat, Isopropylmyristat (IPM), Butylmyristat, Hexylmyristat, Ethylhexylmyristat, Ethylpalmitat, Propylpalmitat, Isopropylpalmitat (IPP), Butylpalmitat, Hexylpalmitat, Ethylstearat, Propylstearat, Isopropylstearat (IPS), Butylstearat, Ethylisostearat, Propylisostearat, Isopropylisostearat (IPIS), Butylisostearat, Ethyloleat, Propyloleat, Isopropyloleat (IPO), Butyloleat, Diethylsuccinat, Dipropylsuccinat, Diisopropylsuccinat, Dibutylsuccinat, Dihexylsuccinat, Diethylhexylsuccinat, Diethylglutarat, Dipropylglutarat, Diisopropylglutarat, Dibutylglutarat, Dihexylglutarat, Diethyladipat, Dipropyladipat, Diisopropyladipat, Dibutyladipat, Dihexyladipat, Diethylsebacinat, Dipropylsebacinat, Diisopropylsebacinat, Dibutylsebacinat und Dihexylsebacinat.

Der erste Erfindungsgegenstand ist dadurch gekennzeichnet, daß Zubereitung (A) als Öl mindestens einen Ester aus C₃-C₄-Monoalkanol mit einer Mono- oder Dicarbonsäure aus der Gruppe, die gebildet wird aus Isopropyllaurat (IPL), Butyllaurat, Isopropylmyristat (IPM), Butylmyristat, Isopropylpalmitat (IPP), Butylpalmitat, Isopropylstearat (IPS), Butylstearat, Isopropylisostearat (IPIS), Butylisostearat, Isopropyloleat (IPO), Butyloleat, Diisopropylsuccinat, Dibutylsuccinat, Diisopropylglutarat, Dibutylglutarat, Diisopropyladipat, Dibutyladipat, Diisopropylsebacinat und Dibutylsebacinat enthält.

Besonders bevorzugte Carbonsäureester werden daher ausgewählt. Besonders bevorzugt sind Isopropylmyristat (IPM), Isopropylpalmitat (IPP) und Dibutyladipat. Diese Verbindungen sind kommerziell erhältlich und werden unter anderem unter den Handelsnamen Crodamol IPM, Stepan IPM oder Lexol IPM NF bzw. Rilanit IPP oder Nikkol IPP sowie Cetiol B (Dibutyladipat) vertrieben.

Unabhängig von der Art des bzw. der ansonsten eingesetzten Öls bzw. Öle sind die erfindungsgemäßen Mittel dadurch gekennzeichnet, daß Zubereitung (A) - bezogen auf ihr Gewicht - 2,5 bis 20 Gew.-% Isopropylmyristat enthält.

Bevorzugte erfindungsgemäße Mittel sind dadurch gekennzeichnet, daß Zubereitung (A) - bezogen auf ihr Gewicht - 3,5 bis 15 Gew.-%, weiter bevorzugt 4 bis 10 Gew.-%, besonders bevorzugt 4,5 bis 7,5 Gew.-% und insbesondere 5 bis 7 Gew.-% Isopropylmyristat enthält.

Die erfindungsgemäßen Mittel können in der Zusammensetzung (A) als Öl auch einen oder mehrere Fettalkohole enthalten, wobei Fettalkohole mit 12 bis 29 Kohlenstoffatomen bevorzugt sind. Als Fettalkohole (Fatal) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die ein Gemisch von unterschiedlichen Fettalkoholen darstellen. Solche Substanzen sind beispielsweise unter den Bezeichnungen Stenol®, z.B. Stenol® 1618 oder Lanette®, z.B. Lanette® O oder Lorol®, z.B. Lorol® C8, Lorol® C14, Lorol® C18, Lorol® C8-18, HD-Ocenol®, Crodacol®, z.B. Crodacol® CS, Novol®, Eutanol® G, Guerbitol® 16, Guerbitol® 18, Guerbitol® 20, Isofol® 12, Isofol® 16, Isofol® 24, Isofol® 36, Isocarb® 12, Isocarb® 16 oder Isocarb® 24 käuflich zu erwerben. Selbstverständlich können erfindungsgemäß auch
Wollwachsalkohole, wie sie beispielsweise unter den Bezeichnungen Corona®, White Swan®, Coronet® oder Fluilan® käuflich zu erwerben sind, eingesetzt werden.

Besonders bevorzugt sind Zusammensetzungen (A); die - bezogen auf ihr Gewicht - 2,5 bis 20 Gew.-%, vorzugsweise 3,5 bis 15 Gew.-%, weiter bevorzugt 4 bis 12,5 Gew.-%, besonders bevorzugt 4,5 bis 10 Gew.-% und insbesondere 5 bis 8 Gew.-% Tetradecan-1-ol, (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), (9*Z*)-Octadec-9-en-1-ol (Oleylalkohol), - (9*E*)-Octadec-9-en-1-ol (Elaidylalkohol), (9*Z*,12*Z*)-Octadeca-9,12-dien-1-ol (Linoleylalkohol), - Eicosan-1-ol (Eicosylalkohol, Arachylalkohol), Docosan-1-ol (Docosylalkohol, Behenylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Brassidylalkohol ((13*E*)-Docosen-1-ol), Erucylalkohol ((13*Z*)-Docos-13-en-1-ol), oder Mischungen aus zweien oder mehreren dieser Alkohole enthalten.

Ganz besonders bevorzugte erfindungsgemäße Mittel sind dadurch gekennzeichnet, daß Zubereitung (A) - bezogen auf ihr Gewicht - 3,5 bis 15 Gew.-%, weiter bevorzugt 4 bis 12,5 Gew.-%, besonders bevorzugt 4,5 bis 10 Gew.-% und insbesondere 5 bis 8 Gew.-% C₁₂₋₁₈-Fettalkohol(e) enthält.

Unter den Ölen haben sich insbesondere Silikonöle als geeignet erwiesen. Cyclische Silikonöle wie Octamethyltetrasiloxan, Decamethylcyclopentasiloxan, Dodecamethylcyclohexasiloxan sind dabei sehr geeignt, wobei Decamethylcyclopentasiloxan (auch schlicht als Cyclopentasiloxan bezeichnet) ein besonders geeignetes Öl für die erfindungsgemäße Anwendung ist. Besonders bevorzugte erfindungsgemäße Mittel sind dadurch gekennzeichnet, daß Zubereitung (A) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 3 Gew.-% Cyclopentasiloxan enthält.

Mit besonderem Vorzug können auch lineare Silicone als Öl eingesetzt werden, wobei lineare Dimethicone durch die folgende Strukturformel (Si1) dargestellt werden:

(SiR¹₃)-O-(SiR²₂-O-)ₓ-(SiR¹₃) (Si1)

Verzweigte Dimethicone können durch die Strukturformel (Si1.1) dargestellt werden:

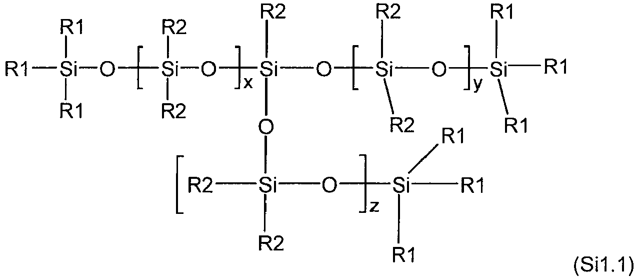

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethicone liegen zwischen 1000 D und 10000000 Dalton. Die Viskositäten liegen zwischen 50 und 10000000 mm2/s gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 100 und 350 mm2/s. Beispielhaft sei hier auf das Produkt "Dow Corning 200 mit einer Viskosität von 50 bis 350 mm2/s verwiesen.

Besonders bevorzugte erfindungsgemäße kosmetische oder dermatologische Zubereitungen sind dadurch gekennzeichnet, dass sie mindestens ein Silikon der Formel (Si1.2)

(CH₃)₃Si-[O-Si(CH₃)₂]ₓ-O-Si(CH₃)₃ (Si1.2),

enthalten, in der x für eine Zahl von 0 bis 100, vorzugsweise von 0 bis 50, weiter bevorzugt von 0 bis 20 und insbesondere 0 bis 10, steht.

Besonders bevorzugte Mittel enthalten kurzkettige Silikone der Formel (Si1.2), in der x für 1, 2, 3, 4 oder 5 steht. Äußerst bevorzugte erfindungsgemäße Mittel sind dadurch gekennzeichnet, daß Zubereitung (A) - bezogen auf ihr Gewicht- 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 3 Gew.-% Octamethyltrisiloxan enthält.

Schließlich können als Öle auch Dimethiconole (Si8) eingesetzt werden. Die Dimethiconole können sowohl linear als auch verzweigt als auch cyclisch oder cyclisch und verzweigt sein. Lineare Dimethiconole können durch die folgende Strukturformel (Si8 - I) dargestellt werden:

(SiOHR¹₂)-O-(SiR²₂-O-)ₓ-(SiOHR¹₂) (Si8-I)

Verzweigte Dimethiconole können durch die Strukturformel (Si8 - II) dargestellt werden:

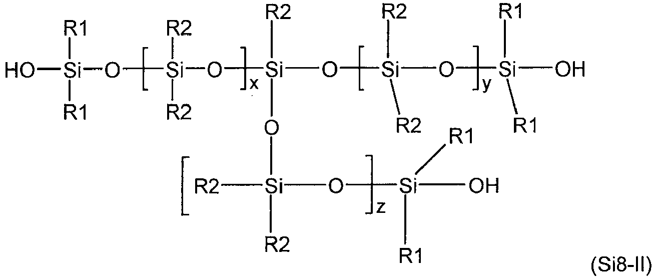

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Die Zahlen x, y und z sind ganze Zahlen und laufen jeweils unabhängig voneinander von 0 bis 50.000. Die Molgewichte der Dimethiconole liegen zwischen 1000 D und 10000000 D. Die Viskositäten liegen zwischen 100 und 350 mm2/s gemessen bei 25 °C mit Hilfe eines Glaskapillarviskosimeters nach der Dow Corning Corporate Testmethode CTM 0004 vom 20. Juli 1970.

Erfindungsgemäß besonders bevorzugte Mittel sind dadurch gekennzeichnet, daß Zubereitung (A) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 3 Gew.-% Dimethiconol(e) enthält.

Unabhängig von der Art der eingesetzten Silikon(e) und davon, ob nur eines oder mehrerer verschiedene Silikone eingesetzt werden, sind erfindungsgemäße Mittel bevorzugt, deren Gesamtmenge an Silikonen in den genannten Grenzen liegt. Äußerst bevorzug5te Mittel sind dadurch gekennzeichnet, daß Zubereitung (A) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 3 Gew.-% Silikon(e) enthält.

Die Zubereitung (A) enthält ferner 1 bis 10 Gew.-% kationische(s) Tensid(e).

Bevorzugte erfindungsgemäße Zusammensetzungen sind dadurch gekennzeichnet, daß die Zubereitung (A) 0,5 bis 20 Gew.-%, vorzugsweise 1 bis 15 Gew.-%, weiter bevorzugt 1,5 bis 10 Gew.-%, besonders bevorzugt 2 bis 7,5 Gew.-% und insbesondere 2,5 bis 5 Gew.-% quartäre Ammoniumverbindung(en) aus der Gruppe
i) der Tetraalkylammoniumhalogenide und/oder
ii) der Esterquats und/oder
iii) der quarternären Imidazoline der Formel (Tkat2) 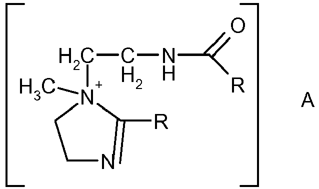 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amine und/oder kationisierten Amine und/oder
v) Poly(methacryloyloxyethyltrimethylammoniumverbindungen) und/oder;
vi) quaternisierten Cellulose-Derivaten, insbesondere Polyquaternium 10, Polyquaternium-24, Polyquaternium-27, Polyquaternium-67, Polyquaternium-72, und/oder
vii) kationische Alkylpolyglycoside und/oder
viii) kationisiertem Honig und/oder
ix) kationischen Guar-Derivaten und/oder
x) polymeren Dimethyldiallylammoniumsalzen und deren Copolymeren mit Estern und Amiden von Acrylsäure und Methacrylsäure, insbesondere Polyquaternium-7 und/oder
xi) Copolymeren des Vinylpyrrolidons mit quaternierten Derivaten des Dialkylaminoalkylacrylats und -methacrylats, insbesondere Polyquaternium-11 und/oder
xii) Vinylpyrrolidon-Vinylimidazoliummethochlorid-Copolymeren, insbesondere Polyquaternium-16 und/oder
xiii) quaterniertem Polyvinylalkohol und/oder
xiv) Polyquaternium-74,
xv) kationischen Alkyloligoglucosiden und/oder
xvi) Polyquaternium-71
sowie deren Mischungen enthalten.

Esterquats gemäß der Formel (Tkat1-2) sind die erste Gruppe der quaternären Ammoniumverbindungen.

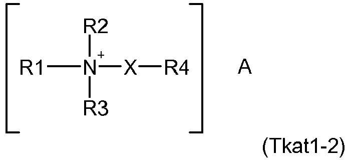

Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- X - R4), mit der Maßgabe, dass höchstens 2 der Reste R1, R2 oder R3 für diesen Rest stehen können:
   Der Rest -(X - R4) ist mindestens 1 bis 3 mal enthalten.

Hierin steht X für:
1) -(CH2)n- mit n = 1 bis 20, vorzugsweise n = 1 bis 10 und besonders bevorzugt n = 1 - 5, oder
2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl,
3) eine Hydroxyalkylgruppe mit ein bis vier Kohlenstoffatomen, welche verzweigt oder unverzweigt sein kann, und welche mindestens eine und höchstens 3 Hydroxygruppen enthält. Beispiele für -X- sind: -CHOH, -CHCH₂OH, -CH₂CHOH, -COHCHOH, -CHOHCOH, -CHCHOHCH₃, -CH₂COHCH₃, -CH₂CHOHCH₂-, -C(CH₂OH)₂, -CH₂CHOHCH₂OH, -CH₂CH₂CHOH, -CH₂COHCH₃ und Hydroxybutylreste, wobei die Bindung von -X- zu R4 von der freien Valenz des betreffenden Kohlenstoffatom ausgeht
und R4 steht für:
1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann,
und A steht für ein physiologisch verträgliches organisches oder anorganisches Anion und wird an dieser Stelle stellvertretend für alle auch im Folgenden beschriebenen Strukturen definiert. Das Anion aller beschriebenen kationischen Verbindungen ist ausgewählt aus den Halogenidionen, Fluorid, Chlorid, Bromid, lodid, Sulfaten der allgemeinen Formel RSO₃⁻, worin R die Bedeutung von gesättigtem oder ungesättigtem Alkylresten mit 1 bis 4 Kohlenstoffatomen hat, oder anionischen Reste organischer Säuren wie Maleat, Fumarat, Oxalat, Tartrat, Citrat, Lactat oder Acetat.

Solche Produkte werden beispielsweise unter den Warenzeichen Rewoquat®, Stepantex®, Dehyquart®, Armocare® und Akypoquat® vertrieben. Die Produkte Armocare® VGH-70, Dehyquart® F-75, Dehyquart® C-4046, Dehyquart® L80, Dehyquart® F-30, Dehyquart® AU-35, Rewoquat® WE18, Rewoquat® WE38 DPG, Stepantex® VS 90 und Akypoquat® 131 sind Beispiele für diese Esterquats.

Weitere erfindungsgemäß besonders bevorzugte Verbindungen der Formel (Tkat1-2) zählen zur Formel (Tkat1-2.1), den kationischen Betainestern.

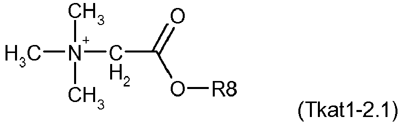

R8 entspricht in seiner Bedeutung R7.

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

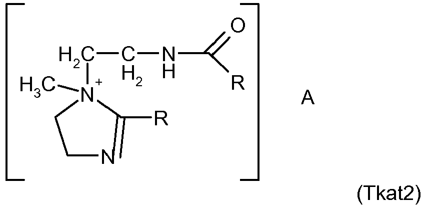

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCII - Bezeichnungen Quaternium-27, Quaternium-72, Quaternium-83 und Quaternium-91 erhältlich. Höchst bevorzugt ist erfindungsgemäß Quaternium-91.

Kationische Tenside der Formel (Tkat1-1) sind die dritte Gruppe bevorzugter quarternärer Ammoniumverbindungen.

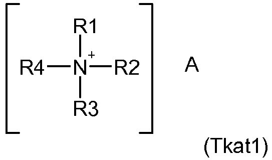

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Besonders bevorzugt sind Verbindungen mit mindestens einem Cetyl- oder Behenylrest im Molekül. Höchst bevorzugt sind Cetyltrimethylammonium und Behenyltrimethylammoniumsalze, allerhöchst bevorzugt Cetyltrimethylammoniumchlorid und Behenyltrimethylammoniumchlorid.

Äußerst bevorzugte kosmetische Zusammensetzungen sind dadurch gekennzeichnet, daß Zubereitung (A) - bezogen auf ihr Gewicht - 0,5 bis 20 Gew.-%, vorzugsweise 1 bis 15 Gew.-%, weiter bevorzugt 1,5 bis 10 Gew.-%, besonders bevorzugt 2 bis 7,5 Gew.-% und insbesondere 2,5 bis 5 Gew.-% C₁₂₋₂₄-Alkyl-ttrimethylammoniumsalz(e) enthält.

Die letzte Gruppe quarternärer Ammoniumverbindungen sind Amine und/oder kationisierte Amine, insbesondere Amidoamine und/oder kationisierte Amidoamine. In einer besonders bevorzugten Ausführungsform der Erfindung enthalten die erfindungsgemäßen Mittel neben mindestens einer weiteren der quarternären Ammoniumverbindungen mindestens ein Amin und/oder kationisiertes Amin, insbesondere ein Amidoamin und/oder ein kationisiertes Amidoamin mit den folgenden Strukturformeln:

R1-NH-(CH₂)ₙ-N⁺R²R³R⁴ A (Tkat3)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und
R2, R3 und R4 jeweils unabhängig voneinander
1) Wasserstoff oder
2) ein Alkylrest mit 1 bis 4 C-Atomen, welcher gleich oder verschieden, gesättigt oder ungesättigt sein kann, und
3) eine verzweigte oder unverzweigte Hydroxyalkylgruppe mit ein bis 4 Kohlenstoffatomen mit mindestens einer und höchstens drei Hydroxygruppen beispielsweise -CH₂OH, -CH₂CH₂OH, -CHOHCHOH, -CH₂CHOHCH₃, -CH(CH₂OH)₂, -COH(CH₂OH)₂, -CH₂CHOHCH₂OH, -CH₂CH₂CH₂OH und Hydroxybutylreste, und
A ein Anion wie zuvor beschrieben und
n eine ganze Zahl zwischen 1 und 10 bedeuten.

Bevorzugt wird eine Zusammensetzung, in welcher das Amin und/oder das quaternisierte Amin gemäß allgemeiner Formeln (Tkat3) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen. Als Beispiele hierfür kommen Lanolin, Bienen-oder Candellilawachse in Betracht.

Bevorzugt sind auch solche Amidoamine und/oder quaternisierte Amidoamine, in denen R2, R3 und/oder R4 in der Formel (Tkat3) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in der allgemeinen Formel (Tkat8) ist eine ganze Zahl zwischen 2 und 5.

Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine.

Beispiele für derartige erfindungsgemäße Handelsprodukte sind Witcamine® 100, Incromine® BB, Mackine® 401und andere Mackine® -Typen, Adogen® S18V, und als permanent kationische Aminoamine: Rewoquat® RTM 50, Empigen® CSC, Swanol® Lanoquat DES-50, Rewoquat® UTM 50, Schercoquat® BAS, Lexquat® AMG-BEO, oder Incroquat® Behenyl HE.

Zubereitung (B) enthält als wesentlichen Inhaltsstoff - bezogen auf ihr Gewicht - mindestens 50 Gew.-% Polyethylenglycol(e) der Formel (I) 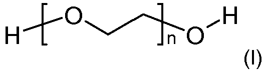 mit n = 6, 7, 8, 9 oder 10

Diese Verbindungen PEG-6, PEG-7, PEG-8, PEG-9 und PEG-10 führen bei Vermischen der Zubereitungen (A) und (B) und bei der Applikation zu einem leichten Wärmeeffekt, der zu einer verbesserten Wahrnehmung der Produktleistung führt und die Pflegeffekte der erfindungsgemäßen Mittel verstärkt.

Bevorzugte erfindungsgemäße Mittel sind dadurch gekennzeichnet, daß Zubereitung (B) - bezogen auf ihr Gewicht - mindestens 60 Gew.-%, vorzugsweise mindestens 70 Gew.-%, weiter bevorzugt mindestens 80 Gew.-%, besonders bevorzugt mindestens 90 Gew.-% und insbesondere mindestens 95 Gew.-% Polyethylenglycol(e) der Formel (I) enthält.

Ganz besonders bevorzugte Mittel enthalten 42,5 bis 67,5 Gew.-%, vorzugsweise 45 bis 65 Gew.-%, weiter bevorzugt 47,5 bis 62,5 Gew.-% und insbesondere 50 bis 60 Gew.-% PEG-7, PEG-8, PEG-9 oder Mischungen von zwei oder allen dreien dieser drei Verbindungen

Äußerst bevorzugte erfindungsgemäße Mittel sind dadurch gekennzeichnet, daß Zubereitung (B) - bezogen auf ihr Gewicht - mindestens 60 Gew.-%, vorzugsweise mindestens 70 Gew.-%, weiter bevorzugt mindestens 80 Gew.-%, besonders bevorzugt mindestens 90 Gew.-% und insbesondere mindestens 95 Gew.-% PEG-8 (n = 8 in der Formel (I)) enthält.

Die erfindungsgemäßen Mittel können darüber hinaus sowohl in der Zubereitung (A) als auch in der Zubereitung (B) oder auch in beiden Zubereitungen weitere Inhaltsstoffe enthalten, die z.B. weitere Pflegeffekte bewirken oder die Applizierbarkeit verbessern. Nachfolgend wird von erfindungsgemäßen Mitteln gesprochen, womit die anwendungsbereite Mischung der Zubereitungen (A) und (B) gemeint ist. Es ist generell bevorzugt, die weiteren Inhaltsstoffe überwiegend in der Zubereitung (A) einzusetzen, d.h. wenn die erfindungsgemäßen Mittel einen entsprechenden Inhaltsstoff enthalten, stammt dieser vorzugsweise aus der Zubereitung (A).

Die Zubereitungen (A) und (B) können im beliebigen Verhältnis zueinander gemischt werden, wobei ein gewichtsgleiches oder annähernd gewichtsgleiches Vermischen bevorzugt ist. Wenn nachfolgend auf Mengen in den erfindungsgemäßen Mitteln Bezug genommen wird, wird ein gewichtsgleiches Vermischen angenommen. Unter der Prämisse, daß die weiteren Inhaltsstoffe vorzugsweise in der Zubereitung (A) enthalten sind, sind in bevorzugten Ausführungsformen der vorliegenden Erfindung die entsprechend für die erfindungsgemäßen Mittel angegebenen Gew.-%-Werte zu verdoppeln und man erhält auf diese Weise die bevorzugten Gew.-%-Angaben für den jeweiligen Inhaltsstoff in der Zubereitung (A).

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.:
Vitamin B₁ (Thiamin)
Vitamin B₂ (Riboflavin)
Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate. Pantothensäure wird bevorzugt als Derivat in Form der stabileren Calciumsalze und Natriumsalze (Ca-Pantothenat, Na-Pantothenat) in der vorliegenden Erfindung eingesetzt.
Vitamin B₆ (Pyridoxin sowie Pyridoxamin und Pyridoxal).

Die genannten Verbindungen des Vitamin B -Typs insbesondere Vitamin B₃, B₅ und B₆, sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Eine besonders bevorzugte Gruppe von Inhaltsstoffen in den erfindungsgemäßen kosmetischen Zusammensetzungen sind die im folgenden genannten Betaine: Carnitin, Carnitintartrat, Carnitin Magnesiumcitrat, Acetylcarnitin, Betalaine, 1,1-Dimethyl-Prolin, Cholin, Cholinchlorid, Cholinbitartrat, Cholindihydrogencitrat und die in der Literatur als Betain bezeichnete Verbindung N,N,N-trimethylglycin.

Bevorzugt werden Carnitin, Histidin, Cholin sowie Betain verwendet. In einer besonders bevorzugten Ausführungsform der Erfindung wird als Wirkstoff L-Carnitintartrat eingesetzt.

In einer weiteren erfindungsgemäß bevorzugten Ausführungsform enthalten die erfindungsgemäßen Zusammensetzungen Biochinone. In den erfindungsgemäßen Mitteln sind unter geeigneten Biochinonen ein oder mehrere Ubichinon(e) und/oder Plastochinon(e) zu verstehen. Die erfindungsgemäß bevorzugten Ubichinone weisen die folgende Formel auf:

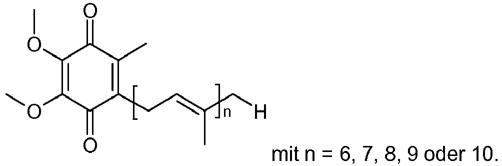

Das Coenzym Q-10 ist hierbei am bevorzugtesten.

Bevorzugte erfindungsgemäße Zusammensetzungen enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten. Erfindungsgemäß bevorzugte kosmetische Mittel sind dadurch gekennzeichnet, daß sie Purin, Adenin, Guanin, Harnsäure, Hypoxanthin, 6-Purinthiol, 6-Thioguanin, Xanthin, Coffein, Theobromin oder Theophyllin enthalten. In haarkosmetischen Zubereitungen ist Coffein am bevorzugtesten.

In einer weiteren bevorzugten Ausführungsform der vorliegenden Erfindung enthält das kosmetische Mittel Ectoin ((*S*)-2-Methyl-1,4,5,6-tetrahydro-4-pyrimidincarbonsäure.

Erfindungsgemäß besonders bevorzugt sind Mittel, die - bezogen auf ihr Gewicht - 0,00001 bis 10,0 Gew.-%, vorzugsweise 0,0001 bis 5,0 Gew.-% und insbesondere 0,001 bis 3 Gew.-% der Wirkstoffe aus der Gruppe, die gebildet wird von Carnitin, Coenzym Q-10, Ectoin, ein Vitamin der B - Reihe, einem Purin und deren Derivaten oder physiologisch vertretbaren Salze enthalten.

Ein ganz besonders bevorzugter Pflegezusatz in den erfindungsgemäßen Haarbehandlungsmitteln ist Taurin. Unter Taurin wird ausschließlich 2-Aminoethansulfonsäure und unter einem Derivat die explizit genannten Derivate des Taurines verstanden. Unter den Derivaten des Taurines werden N-Monomethyltaurin, N,N-Dimethyltaurin, Taurinlysylat, Taurintartrat, Taurinornithat, Lysyltaurin und Ornithyltaurin verstanden.

Besonders bevorzugt sind erfindungsgemäße Mittel, die - bezogen auf ihr Gewicht - 0,0001 bis 10,0 Gew.-%, vorzugsweise 0,0005 bis 5,0 Gew.-%, besonders bevorzugt 0,001 bis 2,0 Gew.-% und insbesondere 0,001 bis 1,0 Gew.-% Taurin und/oder eines Derivates des Taurines enthalten.

Durch die Verwendung von Pflanzenextrakten als Pflegestoffe können die erfindungsgemäßen Haarbehandlungsmittel besonders naturnah und dennoch sehr effektiv in ihrer Pflegeleistung formuliert werden. Gegebenenfalls kann dabei sogar auf ansonsten übliche Konservierungsmittel verzichtet werden. Erfindungsgemäß sind vor allem die Extrakte aus Grünem Tee, Eichenrinde, Brennessel, Hamamelis, Hopfen, Henna, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Baldrian, Wiesenschaumkraut, Quendel, Schafgarbe, Thymian, Melisse, Hauhechel, Huflattich, Eibisch, Meristem, Ginseng, Kaffee, Kakao, Moringa, Ingwerwurzel und ayurvedische Pflanzenextrakte wie beispielsweise Aegle Marmelos (Bilwa), Cyperus Rotundus (Nagar Motha), Emblica Officinalis (Amalki), Morida Citrifolia (Ashyuka), Tinospora Cordifolia (Guduchi), Santalum album, (Chandana), Crocus Sativus (Kumkuma), Cinnamonum Zeylanicum und Nelumbo Nucifera (Kamala), Süßgräser wie Weizen, Gerste, Roggen, Hafer, Dinkel, Mais, die verschiedenen Sorten der Hirse (Rispenhirse, Fingerhirse, Kolbenhirse als Beispiele), Zuckerrohr, Weidelgras, Wiesenfuchsschwanz, Glatthafer, Straußgras, Wiesenschwingel, Pfeifengras, Bambus, Baumwollgras, Lampenputzergräser, Andropogonodeae (Imperata Cylindrica auch Flammengras oder Cogon Gras genannt), Büffelgras, Schlickgräser, Hundszahngräser, Liebesgräser, Cymbopogon (Zitronengras), Oryzeae (Reis), Zizania (Wildreis), Strandhafer, Staudenhafer, Honiggräser, Zittergräser, Rispengräser, Quecken und Echinacea, insbesondere Echinacea purpurea (L.) Moench, aller Arten von Wein sowie Perikarp von Litchie chinensis bevorzugt.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Es hat sich gezeigt, daß bestimmte Proteolipide die Wirkung der erfindungsgemäßen Kombination noch weiter verstärken und die Viskosität sowie deren Langzeitstabilität und die Versprühbarkeit der Zusammensetzung noch weiter verbessern. Als weiteren Inhaltsstoff können die erfindungsgemäßen Mittel mindestens ein Proteolipid der Formel (P-I) enthalten

**R'-X-R"** (P-I),

in der
- R' für einen geradkettigen oder verzweigten, gesättigten oder ungesättigten Kohlenwasserstoffrest mit 11 bis 24 Kohlenstoffatomen steht,
- R" ein Protein, ein Peptid oder ein Proteinhydrolysat bedeutet,
- X für -C(O)O- oder -N⁺(R^{III}₂)R^{IV}- oder -N(R^{III})R^{IV}- oder -C(O)-N(R^{V})R^{VI}- steht,
- R^{III}-(CH₂)ₓ-CH₃ mit x = 0 - 22 bedeutet und
- R^{IV}-CH₂-CH(OH)-CH₂- oder -(CH₂)ₓ- mit x = 0 - 22 bedeutet;
- R^{V} und R^{V} unabhängig voneinander für -H oder -(CH₂)ₓ-CH₃ mit x = 0 - 22 stehen;
mit der Maßgabe, daß R" für Keratin oder ein Keratinhydrolysat steht, wenn X für -C(O)O- steht. Vorzugsweise werden die Proteolipide innerhalb bestimmter Mengen in den erfindungsgemäßen Mitteln eingesetzt. Bevorzugte erfindungsgemäße Kosmetische Mittel enthalten - bezogen auf ihr Gewicht - 0,01 bis 10 Gew.-%, vorzugsweise 0,02 bis 5 Gew.-%, besonders bevorzugt 0,05 bis 2,5 Gew.-%, weiter bevorzugt 0,1 bis 1 Gew.-% und insbesondere 0,15 bis 0,5 Gew.-% Proteolipid(e). Der Rest R" in Formel (P-I) steht für ein Peptid oder ein Protein oder ein Proteinhydrolysat. Wenn X = -C(O)O-, wird R" aus der Gruppe Keratin oder Keratinhydrolysat gewählt.

Bevorzugte Reste R" sind Oligopetide, die mindestens eine Aminosäuresequenz Glu-Glu-Glu 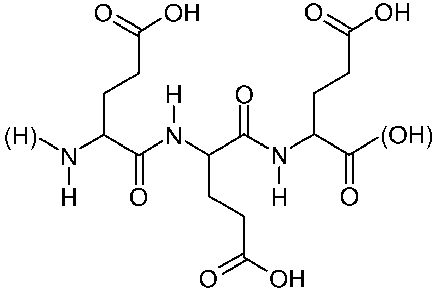 aufweisen, wobei die Amino-Gruppe frei oder protoniert und die Carboxy-Gruppen frei oder deprotoniert vorliegen können.

In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie dargestellt (in der vorstehenden Formel 3) oder aber, daß die Aminosäuresequenz in einem Oligopeptid vorliegt, das noch weitere Anminosäuren umfaßt - je nachdem, wo die weitere(n) Aminosäure(n) gebunden ist/sind, sind die eingeklammerten Bestandteile der o.g. Formel durch den/die weiteren Aminosäurerest(e) ersetzt.

Oligopeptide im Sinne der vorliegenden Anmeldung sind durch Peptid-Bindungen Säureamid-artig verknüpfte Kondensationsprodukte von Aminosäuren, die mindestens 3 und maximal 25 Aminosäuren umfassen

In erfindungsgemäß bevorzugten Haarbehandlungsmitteln der vorstehend beschriebenen Ausführungsform umfaßt das Oligopeptid (= der Rest R") 5 bis 15 Aminosäuren, vorzugsweise 6 bis 13 Aminosäuren, besonders bevorzugt 7 bis 12 Aminosäuren und insbesondere 8, 9 oder 10 Aminosäuren.

Je nachdem, ob weitere Aminosäuren an die Sequenz Glu-Glu-Glu gebunden sind und je nach Art dieser Aminosäuren sowie in Abhängigkeit von der Auswahl der Reste R' und ggf. R^{III} und R^{IV} kann die Molmasse des in den erfindungsgemäßen Mitteln enthaltenen Proteolipids variieren. Erfindungsgemäß bevorzugte Kosmetische Mittel sind dadurch gekennzeichnet, daß das Proteolipid eine Molmasse von 1000 bis 30000 Da, vorzugsweise von 1250 bis 25000 Da, besonders bevorzugt von 1500 bis 20000 Da und insbesondere von 2000 bis 15000 Da aufweist.

Als Rest R" werden vorzugsweise Oligopeptide eingesetzt, die nicht allein aus den drei Glutaminsäuren bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind. So ist es bevorzugt, wenn der Rest R" der in den erfindungsgemäßen Mitteln eingesetzten Proteolipide kein Methionin enthält. Weiter bevorzugt ist es, wenn der Rest R" der in den erfindungsgemäßen Mitteln eingesetzten Proteolipide kein Cystein und/oder Cystin enthält. Weiter bevorzugt ist es, wenn der Rest R" der in den erfindungsgemäßen Mitteln eingesetzten Proteolipide keine Asparaginsäure und/oder Asparagin enthält. Weiter bevorzugt ist es, wenn der Rest R" der in den erfindungsgemäßen Mitteln eingesetzten Proteolipide kein Serin und/oder Threonin enthält.

Demgegenüber ist es bevorzugt, wenn der Rest R" der in den erfindungsgemäßen Mitteln eingesetzten Proteolipide Tyrosin enthält. Weiter bevorzugt ist es, wenn der Rest R" der in den erfindungsgemäßen Mitteln eingesetzten Proteolipide Leucin enthält. Weiter bevorzugt ist es, wenn der Rest R" der in den erfindungsgemäßen Mitteln eingesetzten Proteolipide Isoleucin enthält. Weiter bevorzugt ist es, wenn der Rest R" der in den erfindungsgemäßen Mitteln eingesetzten Proteolipide Arginin enthält. Weiter bevorzugt ist es, wenn der Rest R" der in den erfindungsgemäßen Mitteln eingesetzten Proteolipide Valin enthält.

Als Rest R" besonders bevorzugte Oligopeptide bzw in den bevorzugten Oligopeptiden enthaltene Aminosäuresequenzen werden nachstehend beschrieben:

Ein besonders bevorzugtes Oligopeptid enthält zusätzlich Tyrosin, das vorzugsweise über seine Säurefunktion an die Glu-Glu-Glu-Sequenz gebunden ist. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu aufweist, wobei die Amino-Gruppe frei oder protoniert und die Carboxy-Gruppen frei oder deprotoniert vorliegen können.

Ein weiteres besonders bevorzugtes Oligopeptid enthält zusätzlich Isoleucin, das vorzugsweise über seine Aminofunktion an die Glu-Glu-Glu-Sequenz gebunden ist. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Glu-Glu-Glu-Ile aufweist, wobei die Amino-Gruppe frei oder protoniert und die Carboxy-Gruppen frei oder deprotoniert vorliegen können.

Oligopeptide, die beide vorgenannten Aminosäuren (Tyrosin und Isoleucin) aufweisen, sind erfindungsgemäß bevorzugt. Besonders bevorzugt sind dabei erfindungsgemäße Haarbehandlungsmittel, bei denen das in den Proteolipiden der Formel (I) als Rest R" enthaltene Oligopeptid mindestens eine Aminosäuresequenz Tyr-Glu-Glu-Glu-Ile aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt. Erfindungsgemäß bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg aufweist, wobei die Amino-Gruppen frei oder protoniert und die Carboxy-Gruppen frei oder deprotoniert vorliegen können.

Noch weiter bevorzugte Oligopeptide enthalten zusätzlich Valin, das vorzugsweise an das Arginin gebunden vorliegt. Erfindungsgemäß weiter bevorzugte Kosmetische Mittel sind daher dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val aufweist, wobei die Amino-Gruppen frei oder protoniert und die Carboxy-Gruppen frei oder deprotoniert vorliegen können.

Noch weiter bevorzugte Oligopeptide enthalten zusätzlich Leucin, das vorzugsweise an Valin gebunden vorliegt. Erfindungsgemäß weiter bevorzugte Kosmetische Mittel sind dadurch gekennzeichnet, daß das in den Proteolipiden der Formel (I) als Rest R" enthaltene Oligopeptid mindestens eine Aminosäuresequenz Tyr-Glu-Glu-Glu-Ile-Arg-Val-Leu aufweist, wobei die Amino-Gruppen frei oder protoniert und die Carboxy-Gruppen frei oder deprotoniert vorliegen können. Insbesondere bevorzugte Oligopeptide enthalten zusätzlich Leucin, das vorzugsweise an das Tyrosin gebunden vorliegt. Erfindungsgemäß weiter bevorzugte Kosmetische Mittel sind dadurch gekennzeichnet, daß das in den Proteolipiden der Formel (I) als Rest R" enthaltene Oligopeptid mindestens eine Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können. Zusammenfassend sind insbesondere erfindungsgemäße Kosmetische Mittel bevorzugte, die mindestens ein Proteolipd der Formel (I) enthalten, in der R" mindestens eine Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können.

Wie bereits erwähnt, wird R" aus der Gruppe Keratin oder Keratinhydrolysat gewählt, wenn X = -C(O)O- gilt.

In allen anderen Fällen kann der Rest R" in Formel (P-I) für ein Peptid oder ein Protein oder ein Proteinhydrolysat stehen, wobei Proteinhydrolysate bevorzugt sind. Proteinhydrolysate sind Produktgemische, die durch sauer, basisch oder enzymatisch katalysierten Abbau von Proteinen (Eiweißen) erhalten werden. Erfindungsgemäß können Proteinhydrolysate sowohl pflanzlichen als auch tierischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan® (Cognis), Promois® (Interorgana), Collapuron® (Cognis), Nutrilan® (Cognis), Gelita-Sol® (Deutsche Gelatine Fabriken Stoess & Co), Lexein® (Inolex) und Kerasol® (Croda) vertrieben.

Erfindungsgemäß bevorzugt ist die Verwendung von Proteinhydrolysaten pflanzlichen Ursprungs, z. B. Soja-, Mandel-, Reis-, Erbsen-, Kartoffel- und Weizenproteinhydrolysate. Solche Produkte sind beispielsweise unter den Warenzeichen Gluadin® (Cognis), DiaMin® (Diamalt), Lexein® (Inolex) und Crotein® (Croda) erhältlich.

Vorzugsweise wird unabhängig von der Wahl des X in Formel (P-I) der Rest R" aus Keratin oder keratinhydrolxysaten ausgewählt. Bevorzugte erfindungsgemäße Kosmetische Mittel sind dadurch gekennzeichnet, daß sie mindestens ein Proteolipid der Formel (P-I) enthalten, in der R" für Keratin oder ein Keratinhydrolysat steht.

Insbesondere sind erfindungsgemäße Kosmetische Mittel bevorzugt, die mindestens ein Proteolipd der Formel (P-I) enthalten, in der R^{III}-CH₃ bedeutet und R^{IV} für -(CH₂)ₓ- mit x = 0, 1, 2, 3, 4, 5, 6, 7, 8 steht. Weiter sind besonders bevorzugte erfindungsgemäße Kosmetische Mittel dadurch gekennzeichnet, daß sie mindestens ein Proteolipd der Formel (I) enthalten, in der X für -N⁺(CH₃)₂-CH₂-CH(OH)-CH₂- und R' für -(CH₂)₁₇-CH₃ steht.

Ebenfalls weiter bevorzugte erfindungsgemäße Kosmetische Mittel dadurch gekennzeichnet, daß sie mindestens ein Proteolipd der Formel (P-I) enthalten, in der X für -C(O)-O- und R' für -(CH₂)₁₇-CH₃ steht.

Es hat sich als vorteilhaft erwiesen, zusätzlich zu den Proteolipiden Proteinhydrolysate einzusetzen. Diese verstärken die Wirkung der Proteolipide und werden ihrerseits in ihren Effekten verstärkt. Die Proteinhydrolysate wurden weiter oben als Rest R" detailliert beschrieben. Zusammenfassend sind erfindungsgemäße Kosmetische Mittel bevorzugt, die zusätzlich - bezogen auf ihr Gewicht - 0,01 bis 10 Gew.-%, vorzugsweise 0,05 bis 7 Gew.-%, besonders bevorzugt 0,1 bis 5 Gew.-%, weiter bevorzugt 0,25 bis 2,5 Gew.-% und insbesondere 0,5 bis 2,0 Gew.-% Proteinhydrolysat(e), vorzugsweise Keratinhydrolysat(e) enthalten.

## Patentansprüche

1. Mittel zur Pflege keratinischer Fasern, enthaltend mindestens zwei getrennt voneinander verpackte Zubereitungen (A) und (B) sowie gegebenenfalls eine weitere getrennt von (A) und (B) verpackte Zubereitung (C), die unmittelbar vor der Anwendung zu einer Anwendungsmischung vermischt werden, **dadurch gekennzeichnet, daß**
- Zubereitung (A) eine Haarkur ist, die - bezogen auf ihr Gewicht -
a) 10 bis 60 Gew.-% Wasser,
b) 2,5 bis 20 Gew.-% Isopropylmyristat;
c) 1 bis 10 Gew.-% kationische(s) Tensid(e)
enthält,
wobei Zubereitung (A) maximal 20 Gew.-% Öl(e) enthält
und
- Zubereitung (B) eine fließfähige Zusammensetzung ist, die - bezogen auf ihr Gewicht - mindestens 50 Gew.-% Polyethylenglycol(e) der Formel (I) 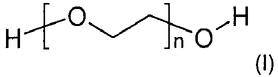 mit n = 6, 7, 8, 9 oder 10
enthält.

2. Mittel nach Anspruch 1, **dadurch gekennzeichnet, daß** Zubereitung (A) - bezogen auf ihr Gewicht - 3,5 bis 15 Gew.-%, weiter bevorzugt 4 bis 10 Gew.-%, besonders bevorzugt 4,5 bis 7,5 Gew.-% und insbesondere 5 bis 7 Gew.-% Isopropylmyristat enthält.

3. Mittel nach einem der Ansprüche 1 oder 2, **dadurch gekennzeichnet, daß** Zubereitung (A) - bezogen auf ihr Gewicht - 3,5 bis 15 Gew.-%, weiter bevorzugt 4 bis 12,5 Gew.-%, besonders bevorzugt 4,5 bis 10 Gew.-% und insbesondere 5 bis 8 Gew.-% Tetradecan-1-ol, (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), (9*Z*)-Octadec-9-en-1-ol (Oleylalkohol), - (9*E*)-Octadec-9-en-1-ol (Elaidylalkohol), (9*Z*,12*Z*)-Octadeca-9,12-dien-1-ol (Linoleylalkohol), - Eicosan-1-ol (Eicosylalkohol, Arachylalkohol), Docosan-1-ol (Docosylalkohol, Behenylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Brassidylalkohol ((13*E*)-Docosen-1-ol), Erucylalkohol ((13*Z*)-Docos-13-en-1-ol), oder Mischungen aus zweien oder mehreren dieser Alkohole enthält.

4. Mittel nach einem der Ansprüche 1 bis 3, **dadurch gekennzeichnet, daß** Zubereitung (A) - bezogen auf ihr Gewicht - 3,5 bis 15 Gew.-%, weiter bevorzugt 4 bis 12,5 Gew.-%, besonders bevorzugt 4,5 bis 10 Gew.-% und insbesondere 5 bis 8 Gew.-% C₁₂₋₁₆-Fettalkohol(e) enthält.

5. Mittel nach einem der Ansprüche 1 bis 4, **dadurch gekennzeichnet, daß** Zubereitung (A) - bezogen auf ihr Gewicht - 0,5 bis 20 Gew.-%, vorzugsweise 1 bis 15 Gew.-%, weiter bevorzugt 1,5 bis 10 Gew.-%, besonders bevorzugt 2 bis 7,5 Gew.-% und insbesondere 2,5 bis 5 Gew.-% C₁₂₋₂₄-Alkyl-ttrimethylammoniumsalz(e) enthält.

6. Mittel nach einem der Ansprüche 1 bis 5, **dadurch gekennzeichnet, daß** Zubereitung (A) - bezogen auf ihr Gewicht - 0,1 bis 10 Gew.-%, vorzugsweise 0,25 bis 7,5 Gew.-%, weiter bevorzugt 0,5 bis 5 Gew.-%, besonders bevorzugt 0,75 bis 4 Gew.-% und insbesondere 1 bis 3 Gew.-% Silikon(e) enthält.

7. Mittel nach einem der Ansprüche 1 bis 6, **dadurch gekennzeichnet, daß** Zubereitung (B) - bezogen auf ihr Gewicht - mindestens 60 Gew.-%, vorzugsweise mindestens 70 Gew.-%, weiter bevorzugt mindestens 80 Gew.-%, besonders bevorzugt mindestens 90 Gew.-% und insbesondere mindestens 95 Gew.-% Polyethylenglycol(e) der Formel (I) enthält.

8. Mittel nach einem der Ansprüche 1 bis 7, **dadurch gekennzeichnet, daß** es- bezogen auf sein Gewicht - 42,5 bis 67,5 Gew.-%, vorzugsweise 45 bis 65 Gew.-%, weiter bevorzugt 47,5 bis 62,5 Gew.-% und insbesondere 50 bis 60 Gew.-% PEG-7, PEG-8, PEG-9 oder Mischungen von zwei oder allen dreien dieser drei Verbindungen enthä#lt.

9. Mittel nach einem der Ansprüche 1 bis 8, **dadurch gekennzeichnet, daß** Zubereitung (B) - bezogen auf ihr Gewicht - mindestens 60 Gew.-%, vorzugsweise mindestens 70 Gew.-%, weiter bevorzugt mindestens 80 Gew.-%, besonders bevorzugt mindestens 90 Gew.-% und insbesondere mindestens 95 Gew.-% PEG-8 (n = 8 in der Formel (I)) enthält.

## Claims

1. An agent for nourishing keratin fibers, containing at least two preparations (A) and (B) that are packaged separately from one another, and optionally a further preparation (C) that is packaged separately from (A) and (B), which preparations are mixed immediately before use to form an application mixture, **characterized in that**
- preparation (A) is a hair masque, which contains, based on the weight thereof,
a) from 10 to 60 wt.% water,
b) from 2.5 to 20 wt.% isopropyl myristate;
c) from 1 to 10 wt.% cationic surfactant(s),
preparation (A) containing a maximum of 20 wt.% oil(s) and
- preparation (B) is a flowable composition, which contains, based on the weight thereof, at least 50 wt.% polyethylene glycol(s) of formula (I) 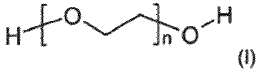 where n = 6, 7, 8, 9 or 10.

2. The agent according to claim 1, **characterized in that** preparation (A) contains, based on the weight thereof, from 3.5 to 15 wt.%, more preferably from 4 to 10 wt.%, particularly preferably from 4.5 to 7.5 wt.%, and in particular from 5 to 7 wt.%, isopropyl myristate.

3. The agent according to one of claims 1 or 2, **characterized in that** preparation (A) contains, based on the weight thereof, from 3.5 to 15 wt.%, more preferably from 4 to 12.5 wt.%, particularly preferably from 4.5 to 10 wt.%, and in particular from 5 to 8 wt.%, tetradecan-1-ol (tetradecyl alcohol, myristyl alcohol), hexadecan-1-ol (hexadecyl alcohol, cetyl alcohol, palmityl alcohol), octadecan-1-ol (octadecyl alcohol, stearyl alcohol), (9*Z*)-octadec-9-en-1-ol (oleyl alcohol), (9*E*)-octadec-9-en-1-ol (elaidyl alcohol), (9*Z*,12*Z*)-octadeca-9,12-dien-1-ol (linoleyl alcohol), eicosan-1-ol (eicosyl alcohol, arachyl alcohol), docosan-1-ol (docosyl alcohol, behenyl alcohol), gadoleyl alcohol ((9*Z*)-eicos-9-en-1-ol), arachidonic alcohol ((5*Z*,8*Z*,11*Z*,14*Z*)-eicosa-5,8,11,14-tetraen-1-ol), brassidyl alcohol ((13*E*)-docosen-1-ol), erucyl alcohol ((13*Z*)-docos-13-en-1-ol), or mixtures of two or more of these alcohols.

4. The agent according to one of claims 1 to 3, **characterized in that** preparation (A) contains, based on the weight thereof, from 3.5 to 15 wt.%, more preferably from 4 to 12.5 wt.%, particularly preferably from 4.5 to 10 wt.%, and in particular from 5 to 8 wt.%, C₁₂₋₁₈ fatty alcohol(s).

5. The agent according to one of claims 1 to 4, **characterized in that** preparation (A) contains, based on the weight thereof, from 0.5 to 20 wt.%, preferably from 1 to 15 wt.%, more preferably from 1.5 to 10 wt.%, particularly preferably from 2 to 7.5 wt.%, and in particular from 2.5 to 5 wt.%, C₁₂₋₂₄ alkyl trimethyl ammonium salt(s).

6. The agent according to one of claims 1 to 5, **characterized in that** preparation (A) contains, based on the weight thereof, from 0.1 to 10 wt.%, preferably from 0.25 to 7.5 wt.%, more preferably from 0.5 to 5 wt.%, particularly preferably from 0.75 to 4 wt.%, and in particular from 1 to 3 wt.%, silicone(s).

7. The agent according to one of claims 1 to 6, **characterized in that** preparation (B) contains, based on the weight thereof, at least 60 wt.%, preferably at least 70 wt.%, more preferably at least 80 wt.%, particularly preferably at least 90 wt.% and in particular at least 95 wt.%, polyethylene glycol(s) of formula (I).

8. The agent according to one of claims 1 to 7, **characterized in that** it contains, based on the weight thereof, from 42.5 to 67.5 wt.%, preferably from 45 to 65 wt.%, more preferably from 47.5 to 62.5 wt.%, and in particular from 50 to 60 wt.%, PEG-7, PEG-8, PEG-9 or mixtures of two or all three of these three compounds.

9. The agent according to one of claims 1 to 8, **characterized in that** preparation (B) contains, based on the weight thereof, at least 60 wt.%, preferably at least 70 wt.%, more preferably at least 80 wt.%, particularly preferably at least 90 wt.% and in particular at least 95 wt.%, PEG-8 (n = 8 in formula (I)).

## Revendications

1. Agent de soin de fibres kératiniques, contenant au moins deux préparations (A) et (B) conditionnées séparément l'une de l'autre ainsi qu'éventuellement une autre préparation (C) conditionnée séparément de (A) et (B), lesquelles sont mélangées à un mélange d'application juste avant l'application, **caractérisé en ce que**
- La préparation (A) est un traitement capillaire contenant - rapportés à son poids -
a) de 10 à 60 % en poids d'eau,
b) de 2,5 à 20 % en poids d'isopropylmyristate ;
c) de 1 à 10 % en poids de tensioactif(s) cationique(s),
dans lequel la préparation (A) contient au plus 20 % en poids d'huile(s) et
- la préparation (B) est une composition fluide contenant, rapporté à son poids, au moins 50 % en poids de polyéthylèneglycol(s) de Formule (I) 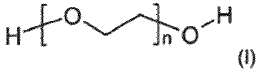 avec n = 6, 7, 8, 9 ou 10.

2. Agent selon la revendication 1, **caractérisé en ce que** la préparation (A) contient - rapporté à son poids - de 3,5 à 15 % en poids, de préférence de 4 à 10 % en poids, de manière particulièrement préférée de 4,5 à 7,5 % en poids et en particulier de 5 à 7 % en poids d'isopropylmyristate.

3. Agent selon la revendication 1 ou 2, **caractérisé en ce que** la préparation (A) contient - rapporté à son poids - de 3,5 à 15 % en poids, de préférence de 4 à 12,5 % en poids, de manière particulièrement préférée de 4,5 à 10 % en poids et en particulier de 5 à 8 % en poids de tétradécan-1-ol, (tétradécylalcool, myristylalcool), d'hexadécan-1-ol (hexadécylalcool, cétylalcool, palmitylalcool), d'octadécan-1-ol (octadécylalcool, stéarylalcool), de (9*Z*)-octadéc-9-en-1-ol (oleylalcool), de (9*E*)-octadéc-9-en-1-ol (élaïdylalcool), de (9*Z*,12*Z*)-octadéca-9,12-dién-1-ol (linoleylalcool), d'éicosan-1-ol (éicosylalcool, arachylalcool), de docosan-1-ol (docosylalcool, béhénylalcool), de gadoléylalcool ((9*Z*)-éicos-9-en-1-ol), d'arachidonalcool ((5*Z*,8*Z*,11*Z*,14*Z*)-éicosa-5,8,11,14-tétraén-1-ol), de brassidylalcool ((13*E*)-docosén-1-ol), d'érucylalcool ((13*Z*)-docos-13-en-1-ol), ou de mélanges d'au moins deux de ces alcools.

4. Agent selon l'une quelconque des revendications 1 à 3, **caractérisé en ce que** la préparation (A) contient - rapporté à son poids - de 3,5 à 15 % en poids, de préférence de 4 à 12,5 % en poids, de manière particulièrement préférée de 4,5 à 10 % en poids et en particulier de 5 à 8 % en poids d'alcool(s) gras en C12-C18.

5. Agent selon l'une quelconque des revendications 1 à 4, **caractérisé en ce que** la préparation (A) contient - rapporté à son poids - de 0,5 à 20 % en poids, de préférence de 1 à 15 % en poids, de manière particulièrement préférée de 1,5 à 10 % en poids, de manière plus particulièrement préférée de 2 à 7,5 % en poids et en particulier de 2,5 à 5 % en poids de sel(s) de triméthylammonium en C₁₂-C₂₄.

6. Agent selon l'une quelconque des revendications 1 à 5, **caractérisé en ce que** la préparation (A) contient - rapporté à son poids - de 0,1 à 10 % en poids, de préférence de 0,25 à 7,5 % en poids, de manière particulièrement préférée de 0,5 à 5 % en poids, de manière plus particulièrement préférée de 0,75 à 4 % en poids et en particulier de 1 à 3 % en poids de silicone(s).

7. Agent selon l'une quelconque des revendications 1 à 6, **caractérisé en ce que** la préparation (B) contient - rapporté à son poids - au moins 60 % en poids, de préférence au moins 70 % en poids, de manière particulièrement préférée au moins 80 % en poids, de manière plus particulièrement préférée au moins 90 % en poids et en particulier au moins 95 % en poids de polyéthylèneglycol(s) de Formule (I).

8. Agent selon l'une quelconque des revendications 1 à 7, **caractérisé en ce qu'**il contient - rapporté à son poids - de 42,5 à 67,5 % en poids, de préférence de 45 à 65 % en poids, de manière particulièrement préférée de 47,5 à 62,5 % en poids, et en particulier de 50 à 60 % en poids de PEG-7, PEG-8, PEG-9 ou de mélanges de deux de ces liants ou de ces trois liants.

9. Agent selon l'une quelconque des revendications 1 à 8, **caractérisé en ce que** la préparation (B) contient - rapporté à son poids - au moins 60 % en poids, de préférence au moins 70 % en poids, de manière particulièrement préférée au moins 80 % en poids, de manière plus particulièrement préférée au moins 90 % en poids et en particulier au moins 95 % en poids de PEG-8 (n = 8 dans la Formule (I)).
